Europäisches Patentamt

European Patent Office

Office européen des brevets

(19)

(11) Veröffentlichungsnummer: **0 148 126**
**A2**

(12) **EUROPÄISCHE PATENTANMELDUNG**

(21) Anmeldenummer: **84810633.2**

(22) Anmeldetag: **17.12.84**

(51) Int. Cl.⁴: **C 07 C 79/37**

(30) Priorität: **23.12.83 CH 6883/83**

(43) Veröffentlichungstag der Anmeldung:
**10.07.85 Patentblatt 85/28**

(84) Benannte Vertragsstaaten:
**CH DE FR GB LI**

(71) Anmelder: **CIBA-GEIGY AG**
**Postfach**
**CH-4002 Basel(CH)**

(72) Erfinder: **Somlo, Tibor, Dr.**
**Florastrasse 8**
**CH-4127 Birsfelden(CH)**

(54) Verfahren zur Isolierung von reinem 1-Nitroanthrachinon aus Nitroanthrachinongemischen.

(57) Beschrieben wird ein Verfahren zur Isolierung von reinem 1-Nitroanthrachinon aus Nitroanthrachinongemischen. Danach wird das rohe 1-Nitroanthrachinon in einem polaren aprotischen Lösungsmittel, dessen Wassergehalt maximal 20 Gew.-% beträgt, suspendiert und die Suspension unter intensiver Durchmischung bei einer Temperatur von −10°C bis +60°C mit einem Alkalimetallhydroxid behandelt. Die Nebenprodukte gehen in Lösung und das reine 1-Nitroanthrachinon wird abfiltriert.

1-Nitroanthrachinon ist ein wichtiges Zwischenprodukt für die Farbstoffsynthese und dient beispielsweise zur Herstellung von Küpenfarbstoffen.

EP 0 148 126 A2

0148126

CIBA-GEIGY AG

Basel (Schweiz)                                    1-14714/=

Verfahren zur Isolierung von reinem 1-Nitroanthrachinon aus Nitroanthrachinongemischen

Die Erfindung betrifft ein Verfahren zur Isolierung von reinem 1-Nitroanthrachinon aus Nitroanthrachinongemischen.

1-Nitroanthrachinon ist ein wichtiges Farbstoffzwischenprodukt, das über weitere Zwischenstufen wie 1-Aminoanthrachinon, 1-Alkyl-aminoanthrachinone, 1-Arylaminoanthrachinone, 1-Halogenanthra-chinone, Dianthrachinonylamine, usw. in zahlreiche Farbstoffe überführt wird. Bedingung für die Verwendbarkeit des 1-Nitroanthrachinons ist jedoch ein sehr hoher Reinheitsgrad. Ungenügende Reinheit des Zwischenproduktes führt nämlich zu Farbstoffen, die in Farbton, Nuancereinheit und Echtheitsniveau den heute gestellten Anforderungen nicht mehr genügen, oder diese nur durch aufwendige Reinigungsoperationen erfüllen.

Ein für die Farbstoffherstellung geeignetes 1-Nitroanthrachinon soll mindestens einen Gehalt von 98,5 $\pm$ 0,5 % aufweisen, d.h. die Summe der gesamten Verunreinigungen darf 2 % nicht übersteigen.

Beim Nitrieren von Anthrachinon zur Herstellung von 1-Nitroanthrachinon entstehen immer beträchtliche Mengen an Nebenprodukten. Durch Wahl des Nitrierungsmediums (Schwefelsäure, Phosphorsäure, Salpetersäure, inerte Lösungsmittel) und der Reaktionsbedingungen (Temperatur, Konzentration, Wassergehalt) lässt sich

- 2 -

die Bildung von Nebenprodukten in gewissen Grenzen beeinflussen,
die Art der Verunreinigungen bleibt jedoch immer gleich und deren
Summe so hoch, dass eine direkte Verwendung des Rohproduktes zur
Farbstoffherstellung nicht möglich ist.

Wird das 1-Nitroanthrachinon z.B. durch Nitrierung von Anthrachinon mit Mischsäure hergestellt (C. Lauth, C.R., 137 661/1903/),
so enthält das Rohprodukt bei vollständiger Nitrierung höchstens
ca. 75 % 1-Nitroanthrachinon, neben 6 bis 7 % 2-Nitroanthrachinon (grösste Einzelverunreinigung), je 4-5 % der 1,5-, 1,6-,
1,7- und 1,8-Dinitroanthrachinone und kleinere Mengen von
Anthrachinon, $\beta,\beta$-Dinitroanthrachinone und Oxidationsprodukte.

In der Literatur sind eine Vielzahl von Reinigungsmethoden für
das 1-Nitroanthrachinon beschrieben. Die meisten dieser Verfahren liefern jedoch ein Produkt, das noch immer bis zu über
4 % durch Nebenprodukte verunreinigt ist. Ferner gelangen in
zahlreichen Verfahren giftige bzw. in der Entsorgung problematische Chemikalien zur Anwendung, wie Hydrazinhydrat oder Sulfite.

In der DE-OS 2 232 446 beispielsweise ist ein Verfahren zur Herstellung von reinem 1-Nitroanthrachinon beschrieben, wonach das
rohe 1-Nitroanthrachinon in einem organischen Lösungsmittel mit
einem primären und/oder sekundären Amin behandelt wird. Durch
Einwirkung des Amins werden die Verunreinigungen chemisch modifiziert und damit löslich im organischen Lösungsmittel und können
auf diese Weise leicht abgetrennt werden. Die Reinheit des nach
diesem Verfahren erhaltenen Produkts reicht jedoch noch nicht aus,
um dessen direkte Weiterverwendung in der Farbstoffherstellung zu
gewährleisten.

Ein Produkt mit der eingangs genannten Reinheit von mindestens 98 %, konnte bislang nur erhalten werden, wenn ein vorgereinigtes, von Anthrachinon und 2-Nitroanthrachinon befreites Rohprodukt der Hochtemperatur-Vakuumrektifikation (Ullmann, DRP 281490/1915/, sowie DE-OS 2.637.689) unterworfen wurde. Eine solche doppelte Reinigung (z.B. Kristallisation und Rektifikation) ist ausserordentlich aufwendig.

Aufgabe der Erfindung war es somit, ein Reinigungsverfahren für rohes 1-Nitroanthrachinon zu entwickeln, das frei ist von den genannten Nachteilen technischer und wirtschaftlicher Natur, und das auf einfache Art und Weise ein Produkt liefert mit einer Reinheit von $\geq$ 98 %.

Gefunden wurde, dass sich 1-Nitroanthrachinon mit der geforderten Reinheit aus Nitroanthrachinongemischen isolieren lässt, wenn man diese in einem polaren aprotischen Lösungsmittel mit einem Wassergehalt bis zu 20 Gew.-% suspendiert, die Suspension unter intensiver Durchmischung bei einer Temperatur von -10° bis +60°C mit einem Alkalimetallhydroxid behandelt und das reine 1-Nitroanthrachinon abtrennt.

Rohes Nitroanthrachinon kann gemäss vorliegendem Verfahren unabhängig von der Nitrierungsmethode, nach der es erhalten wurde, aufgearbeitet werden. Je nach Nitrierungsmethode und Reaktionsbedingungen kann der Gehalt der Rohware an 1-Nitroanthrachinon zwischen 60 und 85 % variieren. Bei den Verunreinigungen handelt es sich in erster Linie um 2-Nitroanthrachinon, Dinitroanthrachinon-Isomere und nicht umgesetztes Anthrachinon. Diese Verbindungen sind stets, wenn auch in unterschiedlicher Menge vorhanden.

Weder die Qualität der gereinigten Ware noch die Trennausbeute werden vom Gehalt der Rohware wesentlich beeinflusst. Der Nitrierungsgrad der Rohware soll jedoch möglichst hoch sein, d.h. sie soll nur noch wenig (1 bis 2 %) nicht nitriertes Anthrachinon enthalten. Die Rohware kann trocken oder feucht, wie sie aus der Nitrierung erhalten wird, eingesetzt werden. Auch ein Restsäure-Gehalt kann toleriert oder durch Erhöhung der Alkalimenge kompensiert werden.

Durch Einwirkung des Alkalimetallhydroxids werden die Verunreinigungen mit hoher Selektivität in eine, im polar aprotischen Lösungsmittel lösliche Form überführt, während das 1-Nitroanthrachinon überraschenderweise im wesentlichen ungelöst in Suspension verbleibt. Auf diese Weise gelingt eine nahezu vollständige Abtrennung des 1-Nitroanthrachinons von den übrigen, in der rohen Nitriermasse vorhandenen Nebenprodukten. Diese werden durch die Alkalibehandlung nur zu einem geringen Teil chemisch modifiziert und können daher unverändert aus dem Lösungsmittel zurückgewonnen und weiterverarbeitet werden.

Als polares aprotisches Lösungsmittel wird bevorzugt Dimethylacetamid, Sulfolan, insbesondere jedoch Dimethylformamid oder Dimethylsulfoxid verwendet. Wesentlich ist, dass der Wassergehalt des Lösungsmittels einen Wert von 20 Gew.-% nicht überschreitet. Einen besonders guten Reinigungseffekt erreicht man, wenn der Wassergehalt des Lösungsmittels 2 bis 15 Gew.-% beträgt. Das jeweils verwendete polare aprotische Lösungsmittel kann vor Eintragen des Nitroanthrachinongemisches durch Zugabe von Wasser entsprechend verdünnt werden; das nötige Wasser kann jedoch auch ganz oder teilweise über das als wässrige Lösung zugesetzte Alkalimetallhydroxid zugegeben werden oder bereits im rohen 1-Nitroanthrachinon enthalten sein, das somit nicht getrocknet werden muss.

Bezogen auf ein Teil Rohprodukt werden vorteilhaft ca. 1 bis 10 Gewichtsteile wasserhaltiges Lösungsmittel verwendet. Das Verhältnis von einem Teil Lösungsmittel zu einem Teil Rohprodukt sollte nicht unterschritten werden, schon allein um
eine Feinverteilung des Nitroanthrachinongemisches zu gewährleisten.
Andererseits sollte ein Verhältnis von 10 Teilen Lösungsmittel auf
ein Teil Rohprodukt nicht überschritten werden, da sonst auch
das 1-Nitroanthrachinon in gewissem Umfang in Lösung geht, was
sich schlussendlich ungünstig auf die Ausbeute auswirkt.

Als Alkalimetallhydroxid, das im vorliegenden Verfahren bevorzugt
in Form einer wässrigen Lösung zur Anwendung gelangt, wird vor
allem Lithium- und insbesondere Natrium- oder Kaliumhydroxid verwendet. Das Alkalimetallhydroxid wird zweckmässigerweise im Ueberschuss (ca. $\geqslant$ 5 % der Theorie) eingesetzt, wobei als stöchiometrische Menge die Menge anzusehen ist, die zur Ueberführung der, im
1-Nitroanthrachinon enthaltenen Verunreinigungen in eine lösliche
Form ausreicht. Bevorzugt verwendet man das Alkalimetallhydroxid
in einem Ueberschuss von 20 bis 100 %. Das Alkalimetallhydroxid
kann dem polar aprotischen Lösungsmittel vor oder auch erst nach
Eintragen des rohen 1-Nitroanthrachinons zugesetzt werden. Insbesondere verwendet man im vorliegenden Verfahren als polar aprotisches
Lösungsmittel Dimethylformamid und als Alkalimetallhydroxid wässrige
Kalilauge.

Durchgeführt wird die Behandlung des im Lösungsmittel suspendierten
Nitroanthrachinongemisches mit dem Alkalimetallhydroxid bei einer
Temperatur von vorzugsweise 0°C bis 50°C, insbesondere 0°C bis 30°C,
wobei die Suspension einer ständigen intensiven Durchmischung unterworfen wird, was vorteilhaft mittels eines mechanischen Dispergiergerätes, beispielsweise eines Scherkopfrührers oder durch Ultraschall
geschieht. Bei der Ultraschall-Dispergierung ist es vorteilhaft, die
Suspension des Nitroanthrachinongemisches im Kavitationsraum eines
Durchflussreaktors zu beschallen. Die Dispergierung kann in jedem
Falle kontinuierlich oder absatzweise erfolgen.

Um die Feinverteilung zu verbessern und zu beschleunigen kann der Nitroanthrachinonsuspension ein Dispergiermittel beigegeben werden. Dabei kommen grundsätzlich anionische, kationische oder auch nichtionische Dispergiermittel in Frage. Selbstverständlich wird man nur solches Dispergiermittel einsetzen, die im alkalischen pH-Bereich stabil sind. Wegen ihrer guten Löslichkeit in organischen Lösungsmitteln sind beispielsweise die, unter der Bezeichnung Tween ® von der Atlas Chemie GmbH vertriebenen nichtionischen Polyäthylenoxid-Addukte gut geeignet.

Ist die Behandlung des dispergierten Nitroanthrachinongemisches mit dem Alkalimetallhydroxid abgeschlossen und sind die Nebenprodukte in Lösung gegangen, so erweist es sich als vorteilhaft, die Suspension vor Abtrennen des reinen 1-Nitroanthrachinons mit einem polaren Lösungsmittel zu verdünnen. Die Suspension lässt sich dann leichter filtrieren und der Nutschkuchen besser trockensaugen. Zur Verdünnung kommen neben Wasser beispielsweise die folgenden Lösungsmittel in Frage: Alkohole, wie Methanol, Aethanol oder Isopropanol, oder auch Ketone wie Aceton oder Methyläthylketon.

Zweckmässigerweise wird das reine 1-Nitroanthrachinon mittels Filtration vom nebenproduktehaltigen Lösungsmittel abgetrennt. Grundsätzlich kommen natürlich auch andere Trennoperationen, wie beispielsweise das Abzentrifugieren in Betracht.

Nach dem Abfiltrieren wird das 1-Nitroanthrachinon mit einem geeigneten Lösungsmittel gewaschen. Ferner ist es im Hinblick auf die Reinheit des Produktes zweckmässig den Filterkuchen mit einem polaren Lösungsmittel auszukochen. Sowohl zum Waschen des isolierten reinen 1-Nitroanthrachinons, wie auch zum Auskochen des Filterkuchens können mit Vorteil die vorgenannten aprotisch polaren Lösungsmittel oder auch die bereits zur Verdünnung der Nitroanthrachinonsuspension eingesetzten polaren Lösungsmittel, verwendet werden. Zum Auskochen des Filterkuchens kommt bevorzugt Dimethylformamid in Frage.

Das erfindungsgemässe Verfahren ermöglicht auf einfache Weise, bei nur geringem apparativen Aufwand, und ohne dass ökologisch bedenkliche Materialien verwendet werden, die Isolierung von reinem 1-Nitroanthrachinon mit einem Gehalt an Nebenprodukten von weniger als 2 %. Die Ausbeute, bezogen auf den Gehalt an 1-Nitroanthrachinon im Rohprodukt beträgt nach diesem Verfahren bis zu 95 %.

Die folgenden Beispiele dienen der Veranschaulichung der Erfindung; Teile bedeuten Gewichtsteile und Prozente Gewichtsprozente.

Beispiel 1: 60 Teile rohes 1-Nitroanthrachinon mit einem Gehalt von 73 % werden während 10 Minuten mittels Ultraschall in einem Gemisch aus 125 Teilen Dimethylformamid und 21 Teilen Wasser suspendiert. Dann werden 8 Teile 50%-ige wässrige Natriumhydroxidlösung zugegeben und die Suspension wird weitere 5 Minuten bei einer Temperatur von 45°C beschallt. Anschliessend giesst man die Suspension in 140 Teile Methanol, kühlt das verdünnte Gemisch auf 20°C, rührt noch eine Stunde und filtriert dann das reine 1-Nitroanthrachinon ab. Das Nutschgut wird nacheinander mit Methanol und Wasser gewaschen und schliesslich mit 80 Teilen Dimethylformamid ausgekocht und bei Raumtemperatur abfiltriert. Nach der Trocknung erhält man 39 Teile 99%-iges 1-Nitroanthrachinon (Trennausbeute 88 % der Theorie).

Beispiel 2: 60 Teile rohes 1-Nitroanthrachinon mit einem Gehalt von 73 % werden in einem Gemisch aus 125 Teilen Dimethylsulfoxid, 21 Teilen Wasser, 10 Teilen 50%-ige wässrige Kaliumhydroxidlösung und einem Teil Polyoxyäthylen-Sorbitan-Laurylester (Tween 20 ®) suspendiert. Die Feinverteilung des rohen 1-Nitro-

- 8 -

anthrachinons erfolgt während 5 Minuten im 30° bis 45°C warmen Lösungsmittelgemisch mit Hilfe eines Scherkopfrührers. Anschliessend giesst man die Suspension in 150 Teile Aceton, rührt das verdünnte Gemisch noch 1 Stunde und filtriert dann das reine 1-Nitroanthrachinon ab. Das Nutschgut wird nacheinander mit Aceton und Wasser gewaschen und schliesslich mit 60 Teilen Aceton ausgekocht. Man erhält auf diese Weise 39 Teile 99,5%-iges Nitroanthrachinon (Trennausbeute > 88 %).

Beispiel 3: 60 Teile rohes 1-Nitroanthrachinon mit einem Gehalt von 73 % werden in einem Gemisch aus 135 Teilen Dimethylformamid und einem Teil Polyoxyäthylen-Sorbitan-Laurylester (Tween 20 ®) unter kräftigem Rühren auf 45°C erhitzt. Dann werden 6 Teile 50%-ige wässerige Natriumhydroxydlösung zugegeben und die Suspension wird weitere 5 Minuten bei 45°C kräftig gerührt. Anschliessend lässt man das Gemisch auf 20 bis 25°C abkühlen, und filtriert das reine 1-Nitroanthrachinon ab. Das Nutschgut wird nacheinander mit Dimethylformamid und Wasser gewaschen. Nach der Trocknung erhält man 38 Teile 98%-iges 1-Nitroanthrachinon. (Trennausbeute 84 % der Theorie).

Beispiel 4: 60 Teile rohes 1-Nitroanthrachinon mit einem Gehalt von 73 % werden in einem Gemisch aus 135 Teilen Dimethylformamid und einem Teil Polyoxyäthylen-Sorbitan-Laurylester (Tween 20 ®) unter kräftigem Rühren auf 45°C erhitzt. Dann werden 6 Teile 50%-ige wässerige Natriumhydroxydlösung zugegeben und die Suspension wird weitere 5 Minuten bei 45°C kräftig gerührt. Anschliessend lässt man das Gemisch abkühlen. Wenn eine Temperatur von 30°C erreicht ist, werden 21 Teile Wasser zugetropft, dann das Gemisch auf 20 bis 25°C gekühlt und das reine 1-Nitroanthrachinon abfiltriert. Das Nutschgut wird nacheinander mit wenig Dimethylformamid und Wasser gewaschen und schliesslich mit 80 Teilen Dimethylformamid ausgekocht. Nach Trocknung erhält man
39 bis 40 Teile 99%-iges 1-Nitroanthrachinon (Trennausbeute 90 % der
Theorie.

Beispiel 5: 60 Teile rohes 1-Nitroanthrachinon mit einem Gehalt von
73 % werden in 135 Teile Dimethylformamid eingerührt. Dann werden
unter weiterem kräftigen Rühren bei 25-30°C 10 Teile 50 %ige wässerige
Kaliumhydroxidlösung zugegeben und die Suspension 30 Minuten weitergerührt. Darauf werden innerhalb 30 Minuten 15 Teile Wasser zugetropft und das reine 1-Nitroanthrachinon abfiltriert. Das Nutschgut
wird nacheinander mit wenig Dimethylformamid und mit Wasser gewaschen
und schliesslich mit 80 Teilen Dimethylformamid ausgekocht. Nach der
Trocknung erhält man 40 Teile 99 %iges 1-Nitroanthrachinon (Trennausbeute 90 % d.Th.)

Vergleichsbeispiel

Die Nacharbeitung des im Beispiel 1 der DE-OS 2 304 233 angegebenen
Reinigungsverfahrens für 1-Nitroanthrachinon liefert ein Produkt,
dessen Zusammensetzung und Reinheitsgrad der folgenden Tabelle
zu entnehmen ist. Zum Vergleich sind die Analysendaten des nach
dem Verfahren gemäss vorliegender Erfindung (Beispiel 1) erhaltenen
Produkts angegeben.

Tabelle

| | Zusammensetzung des rohen 1-Nitroanthra-chinons | gereinigt nach DE-OS 2 304 233 Beispiel 1 | gereinigt nach dem er-findungsgemässen Verfah-ren; Beispiel 1 | |
|---|---|---|---|---|
| | | | ① | ② |
| 1-Nitroanthrachinon | 73,0 % | 80,1 % | 96,8 | 99,3 % |
| Anthrachinon | 1,5 % | 1,6 % | 0,7 | ---- |
| 2-Nitroanthrachinon | 6,2 % | 6,6 % | 0,1 | ---- |
| 1,5-Dinitroanthrachinon | 4,3 % | 2,2 % | 0,9 | 0,6 % |
| 1,6-Dinitroanthrachinon | 4,0 % | 2,6 % | 0,4 | ---- |
| 1,7-Dinitroanthrachinon | 4,5 % | 2,8 % | 0,2 | ---- |
| 1,8-Dinitroanthrachinon | 4,0 % | 2,8 % | 0,6 | ---- |

① vor } Auskochen mit Dimethylformamid
② nach }

Patentansprüche

1. Verfahren zur Isolierung von reinem 1-Nitroanthrachinon aus Nitroanthrachinongemischen, dadurch gekennzeichnet, dass man das durch Nebenprodukte verunreinigte 1-Nitroanthrachinon in einem polaren aprotischen Lösungsmittel mit einem Wassergehalt bis zu 20 Gew.-% suspendiert, die Suspension unter intensiver Durchmischung bei einer Temperatur von -10° bis +60°C mit einem Alkalimetallhydroxid behandelt und das reine 1-Nitroanthrachinon abtrennt.

2. Verfahren gemäss Anspruch 1, dadurch gekennzeichnet, dass man als polares aprotisches Lösungsmittel Dimethylacetamid, Sulfolan, insbesondere Dimethylformamid oder Dimethylsulfoxid verwendet.

3. Verfahren gemäss Anspruch 1, dadurch gekennzeichnet, dass man ein polares aprotisches Lösungsmittel mit einem Wassergehalt von 2 bis 15 Gew.-% verwendet.

4. Verfahren gemäss Anspruch 1, dadurch gekennzeichnet, dass man als Alkalimetallhydroxid Natrium- oder Kaliumhydroxid verwendet.

5. Verfahren gemäss Anspruch 1, dadurch gekennzeichnet, dass man bei einer Temperatur von 0° bis 50°C, insbesondere 0° bis 30°C arbeitet.

6. Verfahren gemäss Anspruch 1, dadurch gekennzeichnet, dass man als polares aprotisches Lösungsmittel Dimethylformamid und als Alkalimetallhydroxid wässrige Kalilauge verwendet.

0148126

7. Verfahren gemäss Anspruch 1, dadurch gekennzeichnet, dass man zur Durchmischung der Nitroanthrachinonsuspension ein mechanisches Dispergiergerät oder Ultraschall verwendet.

8. Verfahren gemäss Anspruch 1, dadurch gekennzeichnet, dass man die Durchmischung der Nitroanthrachinonsuspension in Gegenwart eines Dispergiermittels durchführt.

9. Verfahren gemäss Anspruch 1, dadurch gekennzeichnet, dass man die Nitroanthrachinonsuspension vor Abtrennen des reinen 1-Nitroanthrachinons mit einem polaren Lösungsmittel verdünnt.

10. Verfahren gemäss Anspruch 1, dadurch gekennzeichnet, dass man das abgetrennte 1-Nitroanthrachinon mit einem polaren Lösungsmittel auskocht.

11. Das gemäss dem Verfahren der Patentansprüche 1 bis 10 erhaltene reine 1-Nitroanthrachinon.

12. Verwendung des reinen 1-Nitroanthrachinons gemäss Anspruch 11 zur Herstellung von Anthrachinonfarbstoffen.

FO 7.1 FF/ag*